# EUROPÄISCHE PATENTSCHRIFT

(11) **EP 0 630 662 B1**
(45) Veröffentlichungstag und Bekanntmachung des Hinweises auf die Patenterteilung: **13.09.2000**
(21) Anmeldenummer: 94107399.1
(22) Anmeldetag: 11.05.1994
(51) Int. Cl.: A61N 1/375

(54) **Vorrichtung zum Fixieren eines Elektrodenkabels an einem Gerät**
Device for securing an electrode lead at an apparatus
Dispositif pour fixer un fil d'électrode dans un appareil

(30) Priorität: 01.06.1993 SE 9301856
(43) Veröffentlichungstag der Anmeldung: 28.12.1994
(73) Patentinhaber: Pacesetter AB, 171 95 Solna (SE)
(72) Erfinder: Nyman, Per, S-182 64 Djursholm (SE)
(74) Vertreter: Lettström, Richard Wilhelm

(56) Entgegenhaltungen:
- EP-A- 0 339 877
- US-A- 4 784 141
- US-A- 4 934 366
- US-A- 5 086 773

## Beschreibung

Die Erfindung bezieht sich auf eine Vorrichtung zum Fixieren eines stiftförmigen proximalen Endes eines Elektrodenkabels m einem Anschlussteil eines Herzschrittmachers oder eines Defibrillators, wobei die Fixiervorrichtung ein Federelement umfasst, das etwa senkrecht zur Längsachse des proximalen Endes des Elektrodenkabels, das fixiert werden soll, angeordnet ist und dass das Federelement mit Kleinmitteln zum Fixieren des proximalen Endes des Elektrodenkabels versehen ist, wobei das Federelement teils eine erste Lage einnehmen kann, in der die Klemmittel eine solche Position einnehmen, dass das proximale Ende des Elektrodenkabels die Klemmittel berührungslos passieren kann, und teils eine zweite Lage einnehmen kann, in der die Klemmittel das proximale Ende des Elektrodenkabels angreifen und fixieren.

In der DE-OS 2 518 571 ist eine Fixiervorrichtung beschrieben, die zum Anschliessen mindestens einer Herzschrittmacherelektrode an einen Herzschrittmacher vorgesehen ist. Die Fixiervorrichtung weist eine Schraube auf, die senkrecht zur Längsachse des Elektrodenkabels angeordnet ist und die von oben in das Anschlussteil hineingeschraubt wird, so dass das distale Ende des Elektrodenkabels punktfixiert wird. Um zu vermeiden, dass Körperflüssigkeiten in das Anschlussteil eindringen, ist die Öffnung für die Schraube mit einem Abdichtungsspund versehen. Durch die Schraube und den Abdichtungsspund wird ein verhältnismässig hohes Anschlussteil erhalten. Dies ist nicht wünschenswert, da danach gestrebt wird, dass der Herzschrittmacher die kleinstmögliche Dimension erhalten soll. Ausserdem entsteht durch solche Fixiervorrichtungen für den Operaten das Problem, die Befestigung des Elektrodenkabels am Herzschrittmacher zu sichern. Um sicherzustellen, dass die Schraube, die das Elektrodenkabel fixieren soll, ausreichend hart angezogen ist, aber gleichzeitig die Verbindungsstelle nicht beschädigt, erfolgt das Anziehen mittels eines speziellen Schraubenziehers, der so konstruiert ist, dass er wenigstens zum Teil abbricht, wenn das Soll-Drehmoment erreicht worden ist. Danach wird der Abdichtungsspund mit Hilfe eines weiteren Spezialwerkzeuges appliziert. Dies bedeutet, dass es für den Operaten sehr umständlich ist, das Elektrodenkabel mit dem Herzschrittmacher zu verbinden. Es kommt noch hinzu, dass diese Prozedur in Verbindung mit der Implantation erfolgt, was bedeutet, dass das distale Ende des Elektrodenkabels bereits in das Venensystem des Patienten eingeführt und z.B. im Herz appliziert worden ist. Auch bei einer Reoperation, die notwendig sein kann, wenn eine Herzschrittmacherelektrode defekt ist, oder wenn ein Herzschrittmacher verbraucht ist, ist die beschriebene Fixiervorrichtung schwer zu handhaben, da der Operaten die erwähnten Teile nicht ohne weiteres trennen kann.

In der US-PS 4 913 147 ist ein Anschluss eines Herzschrittmachers, der mit einer sog. schwarzes Loch-Verbindung für das Elektrodenkabel versehen ist, beschrieben. Dies bedeutet, dass das proximale Ende des Elektrodenkabels am Herzschrittmacher ohne Schrauben fixiert und der Fixierbereich ohne Dichtungsspünde abgedichtet wird. Dies erfolgt hier entweder mit Hilfe einer Feder, die im Herzschrittmacheranschluss angeordnet ist und die das stiftförmige proximale Ende des Elektrodenkabels senkrecht zur Längsachse des Stiftes angreifen kann, oder mittels eines Ringes, der im Herzschrittmacheranschluss befestigt ist und durch den das stiftförmige Kabelende hindurchgeschoben wird, wobei der Ring bei einer Fixierung die Form derart ändert, dass er das Kabelende im Anschluss festklemmt. Die Feder und der Ring sind nämlich aus einer Metallegierung, die bei einer ersten Temperatur eine derartige Form aufweist, dass sie ein Hineinschieben des proximalen Endes des Elektrodenkabels in den Anschluss des Herzschrittmachers erlauben und bei einer zweiten Temperatur, in diesem Falle Körpertemperatur, das Elektrodenende in beschriebener Weise fixiert. Der Nachteil einer solchen Elektrodenfixierung ist, dass das Elektrodenkabel erst dann am Herzschrittmacher richtig befestigt ist, wenn der Herzschrittmacher und die Elektrode eingepflanzt sind, d.h., wenn die Feder oder der Ring die Körpertemperatur erreicht haben.

Durch die EP-A 0 048 760 ist ein weiterer Anschluss eines Herzschrittmachers mit einer schwarzes Loch-Verbindung bekannt, bei der die Elektrodenverbindung ohne Hilfe eines Schraubenverbandes oder Dichtungsspünde erfolgt. Der Herzschrittmacheranschluss umfasst eine Schraubenfeder, die mit einem in den Anschluss eingeführten proximalen Ende eines Elektrodenkabels koaxial angebracht ist und entlang eines Teils des Länge des Anschlusses verläuft Die Stirnseiten der Schraubenfeder sind im Anschluss mittels Flanschen von Hülsen, die die Schraubenfeder im Anschlussteil umgeben, festgespannt. Der Innendurchmesser der Schraubenfeder ist etwas kleiner als der Aussendurchmesser des proximalen Endes desjenigen Elektrodenkabels, das an den Herzschrittmacher angeschlossen werden soll. Bei einer Fixierung des Elektrodenendes wird es zunächst zum Teil in die Schraubenfeder hineingeführt. Gleichzeitig wird das Elektrodenende gegen die Windungsrichtung der Schraubenfeder gedreht, wobei die Schraubenfeder expandiert, so dass das ganze Elektrodenende in den Anschluss hingegeführt werden kann. Bei einem applizierten Elektrodenende strebt die Schraubenfeder danach, in ihre ursprüngliche Dimension zurückzugehen, wobei sie das Elektrodenende druckfixiert. Der Herzschrittmacheranschluss umfasst auch ein von der Schraubenfeder getrenntes elektrisches Verbindungsteil. Dieser Herzschrittmacheranschluss ist im Aufbau verhältnismässig kompliziert und, wenn die für eine vorgeschriebene Verbindung erforderliche beschriebene Drehung des Elektrodenendes nicht erfolgt, kann die elektrische Verbindungsoberfläche am Elektrodenende und auch die Schraubenfeder im Herzschrittmacheranschluss zerstört werden.

Durch die US-A-4 784 141 ist eine Vorrichtung zum Fixieren eines stiftförmigen proximalen Endes eines Elektrodenkabels in einem Anschlussteil eines Herzschrittmachers nach dem Oberbegriff des Anspruchs 1 vorbekannt. Das Federelement ist durch eine Schraube zwischen seiner ersten und seiner zweiten Lage verschiebbar ist.

Der Erfindung liegt die Aufgabe zugrunde, eine Fixiervorrichtung der eingangs genannten Art zu schaffen, die im Aufbau sehr einfach und daher in der Herstellung billig ist und die ausserdem in der Handhabung extrem einfach ist, gleichzeitig damit, dass eine gute Fixierung des Elektrodenkabels gegeben ist. Ausserdem soll eine sichere elektrische Verbindung zwischen dem Elektrodenkabel und dem Gerät erreicht werden.

Diese Aufgabe ist erfindungsgemäss dadurch gelöst, dass die Klemmittel an einem radial äusseren Bereich des Federelementes angeordnet sind. Die Fixierung des stiftförmigen Endes des Elektrodenkabels erfolgt nach der Erfindung lediglich dadurch, indem dieses Ende in das Anschlussteil des Herzschrittmachers ohne eine Drehbewegung eingeführt wird. Die Druckfixierung des Kabelendes im Anschlussteil mit Hilfe der Klemmittel ist mindestens genauso wirkungsvoll wie eine Fixierung mittels Schrauben. Der sehr einfache Aufbau der Fixiervorrichtung kann zum grossen Teil ermöglichst werden, indem die beschriebene Lageänderung des Federelements mittels eines Magnets erfolgen kann.

In einer vorteilhaften Ausbildung der Erfindung wird vorgeschlagen, dass das Federelement eine gewölbte Scheibe ist, bei der die Wölbung der Scheibe in der ersten Lage gegen das proximale Ende des Elektrodenkabels und in der zweiten Lage in die entgegengesetzte Richtung zeigt. Durch die beschriebene Form kann das Federelement als ein Schallerzeuger dienen, der ein klickendes Geräusch abgibt, wenn er von der ersten in die zweite Lage schnappt. Wenn die gewölbte Scheibe eine erste beschriebene Lage aufweist, kann das Elektrodenende in das Anschlussteil hineingeführt werden, so dass das Ende wenigstens teilweise das Klemmteil passiert, bis die Stirnseite des Stiftes die Scheibe erreicht. Durch Drücken des Elektrodenendes gegen die Scheibe, so dass die Wölbung deformiert wird, schnappt die Scheibe in die zweite beschriebene Lage, bei der die Wölbung der Scheibe nicht gegen das Elektrodenende zeigt über, wobei die Klemmittel derart aktiviert werden, dass sie gegen das Elektrodenende gedrückt werden und das Ende fixieren. Das klickende Geräusch, das auf diese Weise erzeugt wird, ist eine Bestätigung, dass das Elektrodenende am Gerät fixiert ist. Die Lageänderung der Fixiervorrichtung kann auch mit Hilfe des bereits erwähnten Magneten, der in einer speziellen, später näher beschriebenen Weise appliziert wird, erzielt werden. Bei einem Trennen des Elektrodenkabels vom Gerät kann die gewölbte Scheibe mit Hilfe des Magneten derart aktiviert werden, dass sie wieder die beschriebene erste Lage, bei der das proximale Elektrodenende von der Fixiervorrichtung gelöst werden kann, einnimmt. Der Arzt kann nun wieder ein klickendes Geräusch von der gewölbten Scheibe hören, was anzeigt, dass das Elektrodenende von der Fixiervorrichtung und damit auch vom Gerät gelöst ist.

Eine konstruktiv einfache Ausführung der Erfindung ist dadurch gegeben, dass das Federelement im Profil derart V-förmig ist, dass der Innendurchmesser der Klemmittel kleiner als der Aussendurchmesser des proximalen stiftförmigen Endes des Elektrodenkabels ist. Vor einer Einschiebung des Elektrodenkabels in die Fixiervorrichtung wird das V-förmige Federelement mittels des erwähnten Magnets auseinandergebracht. Wenn nun das Elektrodenende in einer vorgeschriebenen Weise eingeschoben ist, wird der Magnet entfernt, wobei das Federelement in seine ursprüngliche Lage zurückversetzt wird gleichzeitig damit, daß die Klemmittel, die vorzugsweise an den freien Enden des V-förmigen Federelements angebracht sind, das Elektrodenende druckfixieren. Wenn das Elektrodenende vom Gerät getrennt werden soll, wird die V-form des Federelements wieder vom Magneten beeinflusst, so dass die Klemmittel von der Oberfläche des Elektrodenendes entfernt werden.

Im Hinblick auf eine weitere Ausgestaltung der Erfindung wird vorgeschlagen, dass die Klemmittel am Federelement um ein proximales Ende eines applizierten Elektrodenkabels gleich verteilt angebracht sind. Hierdurch wird erreicht, dass das Elektrodenende am Anschlussteil des Gerätes optimal fixiert ist.

In einer weiteren konstruktiv einfachen Ausgestaltung der Erfindung wird vorgeschlagen, dass die Klemmittel kugelförmig ausgebildet sind. Hierdurch kann die Montage der Klemmmittel am Federelement erheblich erleichtert werden, da es ohne Bedeutung ist, welcher Teil der Oberfläche der Klemmittel gegen das Elektrodenende gerichtet werden soll, um dieses Ende zu fixieren. Ausserdem weisen die Klemmittel durch die Form eine verhältnismässig grosse Materialmasse auf, was die Anziehungskraft des Magnets an den Kugeln erhöht, wenn sie aus einem Material bestehen, das von einem Magneten beeinflusst wird.

In einer Weiterbildung der Erfindung wird vorgeschlagen, dass die Klemmittel mit einer im Gerät vorhandenen elektrischen Kontaktstelle elektrisch verbunden sind. Hierdurch wird eine sehr sichere elektrische Kontaktierung zwischen der Elektrode und dem Gerät erhalten.

Eine weitere günstige Ausgestaltung der Erfindung ergibt sich, wenn die Fixiervorrichtung in einem Gehäuse angeordnet ist, das die Form eines abgestumpften Konus hat, dessen Basis am Gerät befestigt ist, wobei der grösste Innendurchmesser des Gehäuses geringfügig grösser als das Federelement in einer ausgestreckten Lage und die Neigung der Innenwand des abgestumpften Konus derart gewählt ist, dass dieser etwa einer Kurve folgt, die die Peripherieoberfläche des Federelements beschreibt, wenn das Federelement von einer ausgestreckten in einer zweiten Lage bewegt wird. Auf diese Weise ist die Fixiervorrichtung, ohne im Gehäuse befestigt zu sein, in diesem fixiert. Dies erleichtert die Montage des Anschlussteils des Geräts.

Im Hinblick auf eine günstige konstruktive Ausgestaltung der Erfindung empfiehlt es sich, dass der mittlere Bereich derjenigen Seite des Federelements, die gegen das Gerät gerichtet ist, mit dem Gerät fest verbunden ist. Diese Ausführungsform ersetzt das erwähnte Gehäuse zum Fixieren des Federelements.

Die Erfindung ist nachfolgend anhand mehrerer in den Zeichnungen dargestellten Ausführungsbeispielen näher erläutert. Es zeigen:
- FIG 1 und 2: Seitenansichten eines schematisch gezeichneten Gerätes zur Abgabe von elektrischen Impulsen mit einer Fixiervorrichtung nach der Erfindung in einem Längsschnitt und in einer passiven und in einer aktiven Fixierlage,
- FIG 3: einen Querschnitt durch die Fixiervorrichtung entlang der Schnittlinie III-III in der FIG 2,
- FIG 4: einen Querschnitt durch die Fixiervorrichtung entlang der Schnittlinie III-III in der FIG 2, aber mit einer weiteren Form des Federelements als die, die in der FIG 3 gezeigt ist und
- FIG 5 bis 7: Seitenansichten eines Gerätes zur Abgabe von elektrischen Impulsen mit Fixiervorrichtungen nach der Erfindung in weiteren Ausführungsformen als die, die in den FIG 1 und 2 gezeigt ist in sowohl passiven Lagen als auch in Fixierlagen.

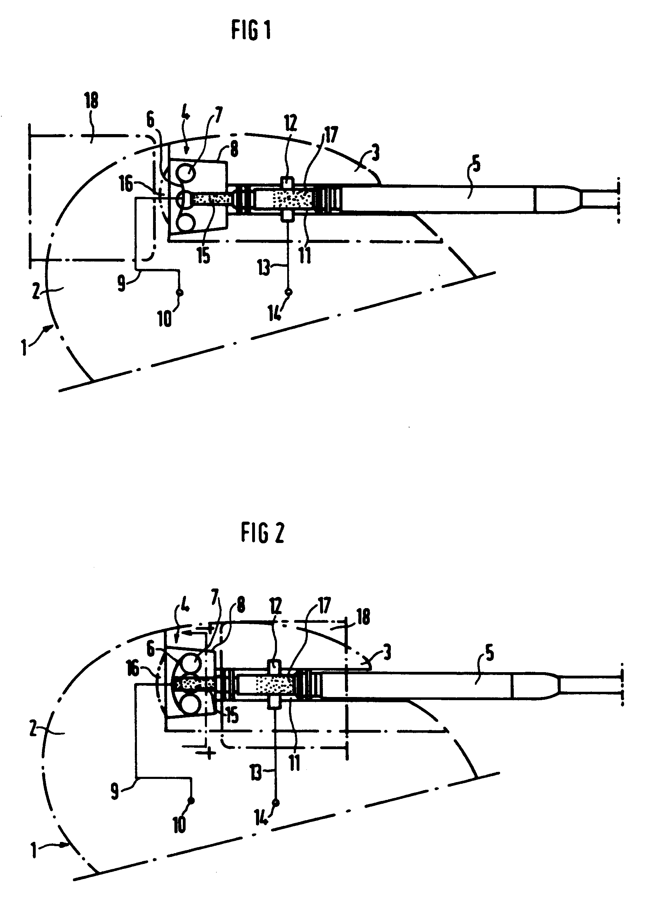

In der FIG 1 sind mit Hilfe von strichpunktierten Linien die Konturen eines Herzschrittmachers 1 gezeigt, der vorwiegend aus einem Stimulationsimpulsgeneratorteil 2 und aus einem Anschlussteil 3 besteht. Im Anschlussteil 3 ist eine Fixiervorrichtung 4 zum Fixieren des stiftförmigen proximalen Endes eines Kabel 5 für eine Herzschrittmacherelektrode angeordnet. Die Fixiervorrichtung 4 umfasst ein Federelement 6, das aus einer gewölbten, vorzugsweise runden Scheibe besteht. Das Federelement bzw. die Scheibe 6 ist mit kugelförmigen Klemmitteln 7 versehen, die am Peripheriebereich der Scheibe 6 gleich verteilt sind. Die Fixiervorrichtung 4 ist in einem Gehäuse 8 angeordnet, das die Form eines abgestumpften Konus hat, dessen Basis am Stimulationsimpulsgeneratorteil 2 befestigt ist. Der grösste Innendurchmesser des Gehäuses 8 ist geringfügig grösser als der Aussendurchmesser der Scheibe 6, wenn diese eine später beschriebene ausgestreckte Lage einnimmt. Die Neigung der Innenwand des abgestumpften Konus, d.h.der Innenwand des Gehäuses 8 ist derart gewählt, dass sie etwa einer Kurve folgt, die die Peripherieoberfläche des Federelements 6 beschreibt, wenn das Federelement 6 von einer ausgestreckten in einer später beschriebenen zweiten Lage bewegt wird. Das Gehäuse 8 dient dazu, das in diesem lose angebrachten Federelement 6 mit dessen kugelförmigen Klemmitteln 7 derart zu fixieren, dass das Federelement 6 immer etwa senkrecht zur Längsachse eines in dem Anschlussteil 3 eingeführten proximalen Endes des Elektrodenkabels 3 angeordnet ist. Die Fixiervorrichtung 4, die aus einem elektrisch leitenden Material besteht, ist über einen Leiter 9 mit einer im Stimulationsimpulsgeneratorteil 2 vorgesehenen elektrischen Verbindungsstelle 10 elektrisch verbunden. Wenn die Herzschrittmacherelektrode bipolar ist, ist im Kanal 11 des Anschlussteils 3 für das proximale Ende des Elektrodenkabels auch ein Verbindungsteil 12 angeordnet, das über einen Leiter 13 mit einer weiteren im Stimulationsimpulsgeneratorteil 2 vorgesehenen elektrischen Verbindungsstelle 14 verbunden ist. Das Anschliessen der Verbindungsstellen 10 und 14 erfolgt in bekannter Weise und wird daher weder näher gezeigt noch beschrieben.

Bevor eine Fixierung des proximalen Endes des Elektrodenkabels 5 erfolgt, ist das Federelement 6 mit Hilfe von später beschriebenen Mitteln in eine erste Lage gebracht worden, in der die Wölbung der Scheibe in Richtung des Kanals 11 zeigt und in der die kugelförmigen Klemmittel 7 eine in der FIG 1 gezeigte Position einnehmen, die es dem stiftförmigen proximalen Ende des Elektrodenkabels 5 ermöglichen, die Klemmittel zu passieren. Danach kann das Elektrodenende 5 durch den Kanal 11 hindurch und in das Gehäuse 8 hineingeführt werden, so dass der Stift des Elektrodenendes 5, das weiterhin mit dem Bezugszeichen 15 bezeichnet wird, gegen die Wölbung der Scheibe 6 anliegt. Bei einer weiteren Verschiebung des Kabelendes 5 drückt der Stift 15 derart gegen die Wölbung, dass die Scheibe 6 zunächst ausgestreckt wird und danach eine zweite Lage einnimmt, in der die Wölbung in eine zur ersten Lage der Wölbung entgegengesetzte Richtung zeigt, wobei die Klemmittel 7 den Stift 15 und damit das Elektrodenkabel 5 am Herzschrittmacher 1, wie es in der FIG 2 dargestellt ist, angreifen und fixieren. Bei einer Verschiebung der Wölbung der Scheibe 6 von einer ersten in eine zweite Lage entsteht ein klickendes Geräusch, das anzeigt, dass das Elektrodenkabel 5 am Herzschrittmacher 1 fixiert ist. Damit die Wölbung der Scheibe 6 mittels des Stiftes 15 von der ersten beschriebenen in die zweite beschriebene Lage verschoben werden kann, ist die Basis des Gehäuses 8 mit einer konkaven Ausnehmung 16 versehen, die nach der Form der Wölbung geformt ist, wenn diese vom Stift 15 wegzeigt und in welcher Ausnehmung 16 die Wölbung mit Hilfe des Stiftes 15 hineingedrückt wird. Die konkave Ausnehmung 16 ist mit einer strichpunktierten Linie dargestellt. Der Stift 15, der elektrisch leitend ist und über einen hier nicht gezeigten Leiter, der im Elektrodenkabel 5 verläuft und z.B. mit einem an dem distalen Ende der Herzschrittmacherelektrode vorgesehenen Elektrodenkopf verbunden ist, ist nunmehr mit dem Stimulationsimpulsgeneratorteil 2 elektrisch verbunden. Auch das Verbindungsteil 12 umschliesst eine Kontaktoberfläche 17 am Elektrodenkabel 5, die über einen weiteren nicht dargestellten Leiter im Elektrodenende 5 mit z.B. einer indifferenten Elektrode verbunden ist.

Das Federelement 6 oder die Scheibe und/oder die Klemmittel 7 bestehen aus einem Material, das durch einen Magneten beeinflusst werden kann. Wenn bei einer Explantation des Herzschrittmachers 1 oder der Herzschrittmacherelektrode 5 das eine Teil vom anderen getrennt werden soll, wird ein Magnet 18, der in der FIG 1 punktgestrichelt dargestellt ist, dicht an die Gehäuseoberfläche des Herzschrittmachers direkt hinter der Fixiervorrichtung 4, von der Einführungsseite des Elektrodenkabels 5 am Anschlussteil 3 gesehen, plaziert. Durch magnetische Kraft werden die kugelförmigen Klemmittel 7 und damit die Scheibe 6 von der zweiten Lage, die in der FIG 2 gezeigt ist, in die erste Lage, die in der FIG 1 dargestellt ist, verschoben, wobei wieder ein klickendes Geräusch zu hören ist, das anzeigt, dass der Stift 15 des Elektrodenendes 5 von der Fixiervorrichtung 4 gelöst ist, so dass das Elektrodenkabel 5 vom Herzschrittmacher 1 getrennt werden kann.

Statt mit Hilfe des Stiftes 15 des Elektrodenendes 5 die Wölbung der Scheibe 6 von einer ersten in eine zweite Lage bei einer Fixierung des Stiftes 15 zu drücken, wie es in der Verbindung mit FIG 1 beschrieben worden ist, kann diese Lageänderung auch mittels des Magneten 18 erfolgen. Der Magnet 18 wird dann dicht gegen die Gehäuseoberfläche des Herzschrittmachers 1 direkt vor der Fixiervorrichtung 4, Seite an Seite und parallel mit dem im Anschlussteil 3 eingeschobenen Elektrodenkabel 5, plaziert. Auf diese Weise werden die kugelförmigen Klemmittel 7 durch die Kraft des Magneten 18 beeinflusst, wobei diese ihrerseits die Wölbung der Scheibe 6 derart beeinflussen, dass sie von einer ersten Lage in eine in der FIG 2 gezeigte zweite Lage, in der die Klemmittel 7 das Elektrodenende 5 fixieren, übergeht. Wenn der Magnet 18 auch bei der Fixierung des Elektrodenendes 5 verwendet wird, ist die konkave Ausnehmung 16 nicht notwendig.

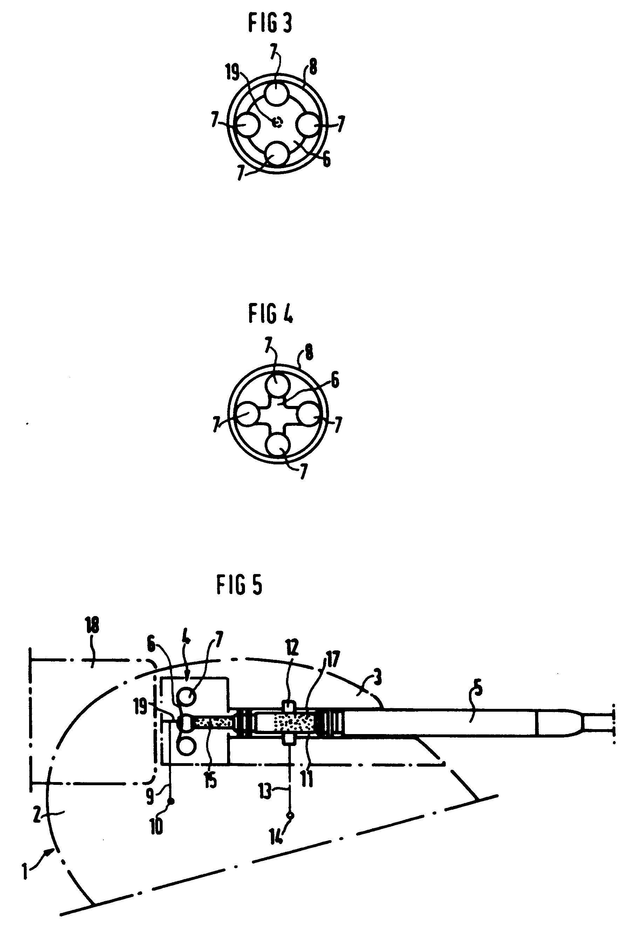

In der FIG 3 ist gezeigt, dass die kugelförmigen Klemmittel 7 an dem äußeren Rand des Federelements 6 bzw. der Scheibe 6 gleich verteilt angebracht sind. Die Anzahl Klemmittel 7 kann grösser oder kleiner sein als die, die in der FIG gezeigt ist.

In der FIG 4 ist gezeigt, dass die Federelemente 6 auch eine weitere, von der runden Form abweichende Form aufweisen können.

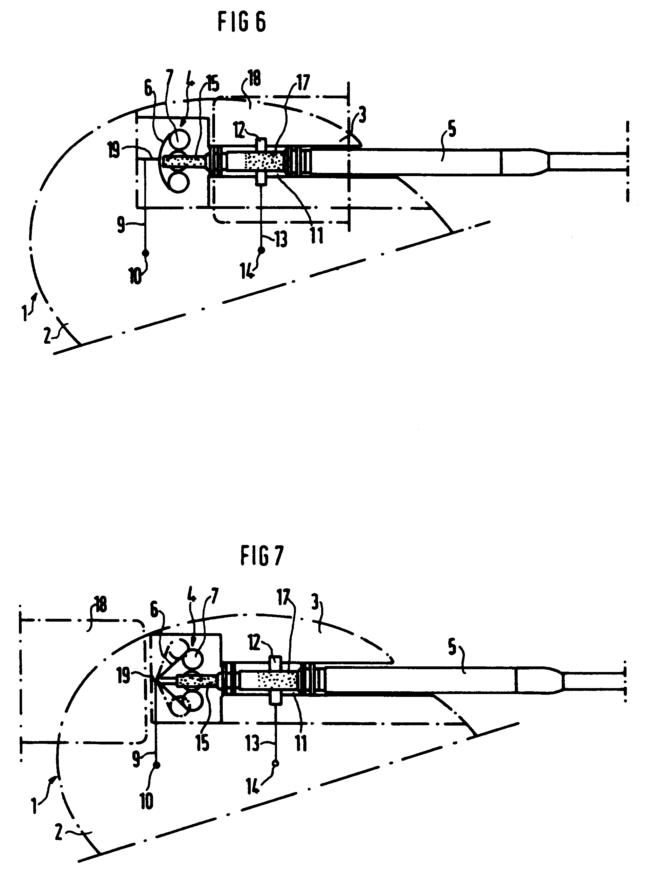

In der FIG 5 ist dargestellt, dass die Fixiervorrichtung 4 mit dem Federelement 6 und die Klemmittel 7 nicht notwendigerweise in einem in den FIG 1 und 2 gezeigten Gehäuse 8 appliziert sein müssen. Das Federelement 6 kann stattdessen über eine stiftförmige Halterung 19 z.B. mit dem Stimulationsimpulsgeneratorteil 2 fest verbunden sein. Die stiftförmige Halterung 19 ist am mittleren Bereich derjenigen Seite des Federelements 6, die gegen das erwähnte Teil 2 gerichtet ist, befestigt. Die Applizierung der Halterung 19 an der Scheibe 6 wird auch in der FIG 3 gezeigt. Die Länge der Halterung 19 ist derart dimensioniert, dass das Federelement 6 auch in einer ersten Lage, d.h. in der Lage, in der die Wölbung des Federlements bzw. der Scheibe 6 gegen das proximale Ende des Elektrodenkabels 5 zeigt, vom Teil 2, in dem die Halterung 19 befestigt ist, frei geht. In der in der FIG 5 und 6 gezeigten Ausführungsform der Fixiervorrichtung 4 wird die beschriebene erste und zweite Lage, wie es in Verbindung mit der FIG 1 und 2 beschrieben ist, lediglich mit Hilfe des Magneten 18 erreicht. Bei einer Verschiebung der kugelförmigen Klemmittel 7 aus einer ersten in eine zweite Lage oder umgekehrt entsteht das bereits beschriebene klickende Geräusch, das darauf hinweist, dass die Herzschrittmacherelektrode 5 in dem einen Fall an der Fixiervorrichtung 4 fixiert und in dem anderen Fall von der Fixiervorrichtung 4 gelöst ist.

In der FIG 7 ist eine Fixiervorrichtung 4 mit einer Form des Federelements 6 gezeigt, die sich von der Form, die in Verbindung mit den bereits gezeigten Ausführungsbeispielen beschrieben worden ist, unterscheidet. In diesem Beispiel ist das Federelement 6 im Profil V-förmig ausgebildet. In einer Draufsicht kann das Federelement vorzugsweise eine Form, die in der FIG 4 gezeigt ist, aufweisen. Vor einer Applizierung eines Elektrodenkabels 5 in dem Anschlussteil 3 wird der Magnet 18, wie es in der FIG gezeigt ist, dicht gegen die Gehäuseoberfläche des Herzschrittmachers 1 direkt hinter die Fixiervorrichtung 4 gelegt. Durch die Kraft des Magneten 18 werden die Klemmittel 7 und das Federelement 6 aus der einen Lage, in der die Klemmittel 7 dicht gegeneinander bzw. annähernd gegeneinander anliegen in eine Lage, in der die Klemmmittel 7 auseinandergehen, gebracht. Die zuletzt erwähnte Lage ist in der FIG 7 durch die strichpunktierte Version der Fixiervorrichtung 4 dargestellt. In dieser Lage kann der Stift 15 am proximalen Ende des Elektrodenkabels 5 in die Fixiervorrichtung 4 hingeingeführt werden, so dass er die Klemmittel 7 passiert. Danach wird das Magnet 18 entfernt, wobei die Klemmittel 7 danach streben, in ihre ursprüngliche Positionen zurückzugehen und somit den Stift 15 zu fixieren. Wenn das Elektrodenkabel 5 vom Herzschrittmacher 1 entfernt werden soll, wird der Magnet 18 in beschriebener Weise wieder gegen die Gehäuseoberfläche appliziert, wobei die Klemmmittel 7 vom Stift 15 entfernt werden. Das Federelement 6 kann direkt oder über eine stiftförmige Halterung 19 am Teil 2 befestigt sein.

Die Fixiervorrichtung 4 ist derart angepasst, dass der Magnet 18 nur dann das Federelement 6 bzw. die Klemmittel 7 beeinflussen kann, wenn dieser in beschriebener Weise dicht gegen die Gehäuseoberfläche des Herzschrittmachers 1 anliegt. Wenn der Herzschrittmacher 1 in einem Patienten implantiert ist und der Magnet 18 dicht gegen den Brustkorb oder den Magen genau vor den Herzschrittmacher 1 gelegt wird, wird die Fixiervorrichtung 4 dagegen nicht beeinflusst, da der Abstand zwischen dieser und dem Magneten 18 dann zu gross ist. Hierdurch kann der Arzt denselben Magneten sowohl für die Fixierung und das Lösen des Elektrodenkabels 5 als auch zum Verändern von Herzschrittmacherfunktionen verwenden gleichzeitig damit, dass es gewährleistet ist, dass übliche Magnete, die bis heute verwendet worden sind, die Fixiervorrichtung nicht unabsichtlich öffnen können.

Die Fixiervorrichtung 4 kann selbstverständlich auch in Verbindung mit einem Defibrillierungsgerät verwendet werden. Der Vorteil mit dieser Fixiervorrichtung gemäss der Erfindung ist, dass das Anschlussteil an das Stimulationsimpulsgeneratorteil 2 voll integriert sein kann, was bedeutet, dass das Gehäuse des Herzschrittmachers diese beiden Teile umschliessen kann. Dies ist herstellungstechnisch einfacher, als wenn das Anschlussteil als ein getrenntes Teil an das Gehäuse angeschlossen werden muss, das dann lediglich das Stimulationsimpulsgeneratorteil 2 umfasst. Hierdurch wird ein von Körperflüssigkeiten sehr gutschliessender Herzschrittmacher erhalten. Ausserdem wird durch die Erfindung eine sehr schnelle, sichere und deutliche Fixierung des Elektrodenkabels am Herzschrittmacher und ein genauso schnelles und sicheres Lösen des Elektrodenkabels vom Herzschrittmacher erreicht, bei denen auch diese Momente mittels eines Geräusches angezeigt werden. Die beschriebenen Klemmmittel brauchen nicht notwendigerweise rund zu sein, sondern können selbstverständlich auch weitere Formen wie z.B. eine Konusform aufweisen, bei der die Spitze des Konuses den Stift am Elektrodenende angreift.

### Bezugszeichenliste

- 1: Herzschrittmacher
- 2: Stimulationsimpulsgeneratorteil,Gerät
- 3: Anschlussteil
- 4: Fixiervorrichtung
- 5: Herzschrittmacherelektrode, Elektrodenkabel, Elektrodenende
- 6: Federelement, Scheibe
- 7: Klemmittel
- 8: Gehäuse
- 9, 13: Leiter
- 10, 14: Verbindungsstelle
- 11: Kanal
- 12: Verbindungsteil
- 15: Stift
- 16: Ausnehmung
- 17: Kontaktoberfläche
- 18: Magnet
- 19: Halterung

## Patentansprüche

1. Vorrichtung zum Fixieren eines stiftförmigen proximalen Endes (15) eines Elektrodenkabels (5) in einem Anschlussteil (3) eines Herzschrittmachers (1) oder eines Defibrillators, wobei die Fixiervorrichtung (4) ein Federelement (6) umfasst, das etwa senkrecht zur Längsachse des proximalen Endes des Elektrodenkabels (5), das fixiert werden soll, angeordnet ist, wobei das Federelement (6) mit Klemmitteln (7) zum Fixieren des proximalen Endes des Elektrodenkabels (5) versehen ist, und wobei das Federelement (6) teils eine erste Lage einnehmen kann, in der die Klemmittel (7) eine solche Position einnehmen, dass das proximale Ende des Elektrodenkabels (5) die Klemmittel (7) berührungslos passieren kann, und teils eine zweite Lage einnehmen kann, in der die Klemmittel (7) das proximale Ende des Elektrodenkabels (5) angreifen und fixieren, **dadurch gekennzeichnet**, dass die Klemmittel (7) an einem radial äusseren Bereich des Federelementes (6) angeordnet sind.

2. Vorrichtung nach Anspruch 1, **dadurch gekennzeichnet**, dass das Federelement (6) eine gewölbte Scheibe ist, bei der die Wölbung der Scheibe (6) in der ersten Lage gegen das proximale einzuführende Ende des Elektrodenkabels (5) und in der zweiten Lage in die entgegengesetzte Richtung zeigt.

3. Vorrichtung nach Anspruch 1, **dadurch gekennzeichnet**, dass das Federelement (6) im Profil derart V-förmig ist, dass der Innendurchmesser der Klemmittel (7) kleiner als der Aussendurchmesser des einzuführenden proximalen stiftförmigen Endes des Elektrodenkabels (5) ist.

4. Vorrichtung nach einem der Ansprüche 1 bis 2, **dadurch gekennzeichnet**, dass die Klemmittel (7) am Federelement (6) in dessen Umfangsrichtung gleich verteilt angebracht sind.

5. Vorrichtung nach einem der Ansprüche 1 bis 4, **dadurch gekennzeichnet**, dass die Klemmittel (7) kugelförmig ausgebildet sind.

6. Vorrichtung nach einem der Ansprüche 1 bis 5, **dadurch gekennzeichnet**, dass die Klemmittel (7) mit einer im Herzschrittsmacher (1) oder im Defibrillator vorhandenen elektrischen Verbindungsstelle (10) elektrisch verbunden sind.

7. Vorrichtung nach einem der Ansprüche 1 bis 6, **dadurch gekennzeichnet**, dass das Federelement (6) und/oder die Klemmittel (7) aus einem Material bestehen, das durch einen Magneten beeinflussbar ist.

8. Vorrichtung nach einem der Ansprüche 1, 2 und 4 bis 7, **dadurch gekennzeichnet**, dass die Fixiervorrichtung (4) in einem Gehäuse (8) angeordnet ist, das die Form eines abgestumpften Konus hat, dessen Basis am Herzschrittmacher (1) oder am Defibrillator befestigt ist, wobei der grösste Innendurchmesser des Gehäuses (8) geringfügig grösser als das Federelement (6) in einer ausgestreckten Lage und die Neigung der Innenwand des abgestumpften Konus derart gewählt ist, dass dieser etwa einer Kurve folgt, die die Peripherieoberfläche des Federelements (6) beschreibt, wenn das Federelement (6) von einer ausgestreckten in einer zweiten Lage bewegt wird.

9. Vorrichtung nach einem der Ansprüche 1 bis 7, **dadurch gekennzeichnet**, dass der mittlere Bereich derjenigen Seite des Federelements (6), die gegen den Herzschrittmacher (1) oder den Defibrillator gerichtet ist, mit dem Herzschrittmacher (1) oder dem Defibrillator fest verbunden ist.

## Claims

1. Arrangement for fixing a pin-shaped proximal end (15) of an electrode cable (5) in a connecting portion (3) of a heart pacemaker (1) or a defibrillator, in which the fixing arrangement (4) comprises a spring element (6) which is arranged so as to be substantially perpendicular to the longitudinal axis of the proximal end of the electrode cable (5) that is to be fixed, in which the spring element (6) is provided with clamping means (7) for fixing the proximal end of the electrode cable (5), and in which the spring element (6) can partly occupy a first position in which the clamping means (7) occupy such a position that the proximal end of the electrode cable (5) can pass through the clamping means (7) in a contact-free manner, and can partly occupy a second position in which the clamping means (7) act upon and fix the proximal end of the electrode cable (5), characterised in that the clamping means (7) are arranged on a radially outer region of the spring element (6).

2. Arrangement according to claim 1, characterised in that the spring element (6) is a curved disc, in which case the curvature of the disc (6) in the first position points towards the proximal end of the electrode cable (5) that is to be inserted and in the second position points in the opposite direction.

3. Arrangement according to claim 1, characterised in that the spring element (6) has a V-shaped profile so that the inside diameter of the clamping means (7) is smaller that the outside diameter of the proximal pin-shaped end of the electrode cable (5) that is to be inserted.

4. Arrangement according to one of claims 1 to 2, characterised in that the clamping means (7) are fitted on the spring element (6) so as to be evenly distributed in the latter's peripheral direction.

5. Arrangement according to one of claims 1 to 4, characterised in that the clamping means (7) are formed in a spherical manner.

6. Arrangement according to one of claims 1 to 5, characterised in that the clamping means (7) are electrically connected to an electrical connecting point (10) that is provided in the heart pacemaker (1) or in the defibrillator.

7. Arrangement according to one of claims 1 to 6, characterised in that the spring element (6) and/or the clamping means (7) consist of a material that can be influenced by a magnet.

8. Arrangement according to one of claims 1, 2 and 4 to 7, characterised in that the fixing arrangement (4) is arranged in a housing (8) that has the form of a truncated cone, the base of which is secured to the heart pacemaker (1) or to the defibrillator, with the largest inside diameter of the housing (8) being slightly larger than the spring element (6) in an extended position and with the incline of the inside wall of the truncated cone being selected so that the latter substantially follows a curve that describes the peripheral surface of the spring element (6) when the spring element (6) is moved from an extended position into a second position.

9. Arrangement according to one of claims 1 to 7, characterised in that the central region of that side of the spring element (6) that is directed towards the heart pacemaker (1) or the defibrillator is fixedly connected to the heart pacemaker (1) or to the defibrillator.

## Revendications

1. Dispositif pour immobiliser une extrémité (15) proximale en forme de broche d'un câble (5) d'électrode dans une partie (3) de raccordement d'un stimulateur (1) cardiaque ou d'un défibrillateur, le dispositif (4) d'immobilisation comportant un élément (6) élastique qui est monté à peu près perpendiculairement à l'axe longitudinal de l'extrémité proximale du câble (5) d'électrode que l'on cherche à immobiliser, l'élément (6) élastique étant muni de moyens (7) de serrage pour immobiliser l'extrémité proximale du câble (5) d'électrode, et l'élément (6) élastique pouvant prendre tantôt une première position en laquelle les moyens (7) de serrage prennent une position telle que l'extrémité proximale du câble (5) d'électrode peut passer sans les toucher dans les moyens (7) de serrage et tantôt une deuxième position en laquelle les moyens (7) de serrage saisissent une extrémité proximale du câble (5) d'électrodes et l'immobilise, caractérisé en ce que les moyens (7) de serrage sont montés sur une zone radialement extérieur de l'élément (6) élastique.

2. Dispositif suivant la revendication 1, caractérisé en ce que l'élément (6) élastique est un disque incurvé dans lequel la courbure du disque (6) est tournée vers l'extrémité proximale à introduire du câble (5) d'électrode en la première position et en sens opposé en la seconde position.

3. Dispositif suivant la revendication 1, caractérisé en ce que l'élément (6) élastique est de profil en forme d'un V tel que le diamètre intérieur des moyens (7) de serrage est inférieur au diamètre extérieur de l'extrémité proximale en forme de broche à introduire du câble (5) d'électrode.

4. Dispositif suivant l'une des revendications 1 à 2, caractérisé en ce que les moyens (7 )de serrage sont montés sur l'élément (6) élastique en étant répartis de manière uniforme dans sa direction périphérique.

5. Dispositif suivant l'une des revendications 1 à 4, caractérisé en ce que les moyens (7) de serrage sont conformés en forme de bille.

6. Dispositif suivant l'une des revendications 1 à 5, caractérisé en ce que les moyens (7) de serrage sont reliés de manière conductrice de l'électricité à un point (10) de liaison électrique présent dans le stimulateur (1) cardiaque ou dans le défibrillateur.

7. Dispositif suivant l'une des revendications 1 à 6, caractérisé en ce que l'élément (6) élastique et/ou les moyens (7) de serrage sont en un matériau qui peut être influencé par un aimant.

8. Dispositif suivant l'une des revendications 1, 2 et 4 à 7, caractérisé en ce que le dispositif (4) d'immobilisation est monté dans un boîtier (8) qui a la forme d'un cône tronqué dont la base est fixée au stimulateur (1) cardiaque ou au défibrillateur, le plus grand diamètre intérieur du boîtier (8) étant choisi légèrement plus grand que l'élément (6) élastique en position allongée et la pente de la paroi intérieure du cône tronqué étant choisie, de telle manière que ce dernier suit à peu près une courbe qui décrit la surface périphérique de l'élément (6) élastique quand l'élément (6) élastique est déplacé d'une position allongée à une deuxième position.

9. Dispositif suivant l'une des revendications 1 à 7, caractérisé en ce que la zone médiane de la face de l'élément (6) élastique, qui est tournée vers le stimulateur (1) cardiaque ou le défibrillateur, est reliée de manière fixe au stimulateur (1) cardiaque ou au défibrillateur.
